# EUROPEAN PATENT APPLICATION

(11) **EP 4 760 126 A1**
(43) Date of publication of application: **17.06.2026**
(21) Application number: 24864279.5
(22) Date of filing: 22.07.2024
(51) Int. Cl.: F16H 57/02, A61B 34/37, A61B 34/00, H02K 7/10

(54) **POWER BOX OUTPUT ASSEMBLY, POWER BOX, SLIDE TABLE ASSEMBLY, AND SURGICAL ROBOT**

(30) Priority: 12.09.2023 CN 202311174679
(71) Applicant: Agibot Medtech (Suzhou) Co., Ltd., Suzhou, Jiangsu 215123 (CN)
(72) Inventor: PENG, Cheng, Suzhou, Jiangsu 215123 (CN); XU, Min, Suzhou, Jiangsu 215123 (CN); WANG, Yupu, Suzhou, Jiangsu 215123 (CN); LIN, Size, Suzhou, Jiangsu 215123 (CN)
(74) Representative: Oancea, Andreas Christian
(86) International application number: PCT/CN2024/106742
(87) International publication number: WO 2025/055555

(57) **Abstract**

A power box output assembly, a power box, a slide carriage assembly, and a surgical robot are disclosed. The power box assembly includes a transmission member (1), an output member (2), and an elastic member (3). The transmission member (1) is connected to a motor shaft, and the output member (2) is sleeved on the transmission member (1). The elastic member (3) is disposed between the transmission member (1) and the output member (2), with one end of the elastic member (3) abutting the transmission member (1) and the other end of the elastic member (3) abutting the output member (2). A limiting engagement mechanism is provided between the transmission member (1) and the output member (2) to prevent axial separation and circumferential relative rotation thereof through surface-to-surface contact.

## Description

The present disclosure claims priority to Chinese Patent Application No. 202311174679.1, filed on September 12, 2023, and the entire contents of which are incorporated herein by reference.

### FIELD OF THE INVENTION

The present disclosure relates to the field of medical instruments, and specifically to a power box output assembly, a power box, a slide carriage assembly, and a surgical robot.

### BACKGROUND OF THE INVENTION

Minimally invasive surgery, also known as endoscopic surgery, utilizes endoscopic imaging systems and surgical robots to perform operations inside the patient's body through natural orifices or small incisions. Currently, master-slave surgical robots are widely used in minimally invasive surgery. These robots consist of a master control arm and slave manipulator arms. The master console collects the surgeon's operation signals, which are then processed by the control system to generate control signals for the slave manipulator arms, which then execute the surgical procedures.

### SUMMARY OF THE INVENTION

In one aspect, the present disclosure provides a power box output assembly, which includes a transmission member, an output member, and an elastic member. The transmission member is detachably and fixedly connected to the motor shaft of a motor, and the output member is sleeved on the transmission member. The elastic member is disposed between the transmission member and the output member, with one end of the elastic member abutting the transmission member and the other end of the elastic member abutting the output member to bias the output member away from the transmission member. A limiting engagement mechanism is provided between the transmission member and the output member to prevent axial separation and circumferential relative rotation thereof.

In another aspect, the present disclosure provides a power box, which includes a motor, a housing, and the aforementioned power box output assembly. Multiple motors are disposed within the housing, and the motor shaft of each motor is connected to the transmission member.

In another aspect, the present disclosure provides a slide carriage assembly, which includes a slide carriage and the aforementioned power box. The power box is mounted on the slide carriage, and the slide carriage is provided with a drive mechanism configured to drive the power box to slide linearly on the slide carriage.

In another aspect, the present disclosure provides a surgical robot, which includes a main control arm and a slave manipulator arm, wherein the main control arm and the slave manipulator arm are signal-connected, and the end of the slave manipulator arm is the aforementioned slide carriage assembly. The power box is detachably connected to an instrument box and transmits the rotational motion force of different motors to the instrument box.

By referring to the detailed description of one or more of the following embodiments, those skilled in the art will be able to more fully understand the inventive content of the present disclosure and realize its additional beneficial effects.

### BRIEF DESCRIPTION OF THE DRAWINGS

FIG 1 is a diagram of the power box.
FIG 2 is a diagram showing the structure at section A in FIG 1.
FIG 3 is a diagram of the transmission member.
FIG 4 is a structural diagram showing the location of the mounting holes.
FIG 5 is a structural diagram showing the location of the motor shaft hole.
FIG 6 is a structural diagram of the output member.
FIG 7 is a structural diagram showing the location of the notch.
FIG 8 is a structural diagram showing the location of the top plate.
FIG 9 is an enlarged diagram of region B in FIG 8.
FIG 10 is an enlarged diagram of region E in FIG 2.
FIG 11 is a diagram of the top plate.
FIG 12 is a structural diagram of the support plate.
FIG 13 is a structural diagram of the slide carriage assembly.

### List of reference numerals in the drawings:

1 - Transmission Member
11 - Main Shaft
111 - Motor Shaft Hole
112 - Mounting Hole
12 - First Limiting Ring
121 - Sliding Slot
122 - Limiting Slot
13 - Second Limiting ring
131 - Threaded Hole
2 - Output Member
21 - Limiting Protrusion
22 - Cover
23 - Flange Ring
231 - Positioning Hole
232 - Notch
3 - Elastic Member
4 - Support Post
5 - Top Plate
51 - Third Limiting Ring
52 - Second Limiting Hole
6 - Fixing boss
61 - First Limiting Hole
7 - Fixing bolt
91 - Housing
92 - Motor
10 - Power Box
20 - Slide Carriage

### DETAILED DESCRIPTION OF THE EMBODIMENTS

The technical solutions of the embodiments of the present disclosure will be described with reference to the accompanying drawings.

The description contains numerous specific technical details. However, it should be understood that embodiments of the present disclosure may be implemented without these specific technical details. Such detailed descriptions should not be construed as limiting, and the scope of protection of the present disclosure is defined only by the claims. Elsewhere, well-known structures, circuits, and other details have not been shown in detail to avoid misleading the public about the essential points of the present disclosure.

In the present disclosure, the accompanying drawings illustrate diagrams of several embodiments of the present disclosure. However, the drawings are merely illustrative, and it should be understood that other embodiments or combinations may be used, and changes in mechanical structure, physical composition, electrical aspects, and procedures may be made without departing from the spirit and scope of the present disclosure.

The terminology used herein is for the purpose of describing particular embodiments only and is not intended to limit the present disclosure. Spatially relative terms, such as "below," "lower," "above," "upper," etc., are used for ease of explanation to describe the relationship between one element or feature illustrated in the figures and another element or feature. It should be understood that spatially relative terms are intended to cover different orientations of the device in use or operation other than those depicted in the figures. For example, if the device in the figures is flipped over, then an element described as "below" other elements or features will become "above" other elements or features. Thus, the exemplary term "below" may cover both above and below orientations. The device may be oriented in other ways (e.g., rotated 90° or in other orientation), and the spatially relative descriptive terms used herein shall be interpreted accordingly.

As used herein, "several," the singular form "one," and "the" are intended to include the plural form as well, unless the context otherwise indicates. It should be further understood that the terms "comprising" and/or "including" specify the presence of the stated feature, step, operation, element, and/or component without excluding the presence of one or more other features, steps, operations, elements, components, and/or combination thereof.

The term "object" generally refers to a component or a group of components. Throughout the description and claims, the terms "object," "component," "portion," "part," and "member" may be used interchangeably.

The terms "instrument," "surgical instrument," and "surgical device" are used herein to describe medical devices configured for insertion into a patient and for performing surgical or diagnostic procedures, including end effectors. End effectors may be surgical tools associated with one or more surgical tasks, such as forceps, needle holders, scissors, bipolar cauterizers, tissue stabilizers or retractors, clamp applicators, anastomosis devices, imaging devices (e.g., endoscopes or ultrasound probes), and the like. Some instruments used in embodiments of the present disclosure further provide articulated supports (sometimes referred to as "wrists") for the surgical tool, allowing the position and orientation of the end effector to be manipulated relative to the instrument axis with one or more mechanical degrees of freedom. Further, many end effectors include functional and mechanical degrees of freedom, such as jaws that open or close or a knife that translates along a path. Instruments may also contain permanently stored information or stored information updatable by the surgical system (e.g., on a PCBA board within the instrument). Accordingly, the system may provide one-way or two-way communication between the instrument and one or more system components.

The term "engage/engagement" may be broadly understood as any situation in which two or more objects are connected in a manner that allows the engaged objects to operate in combination with each other. It should be noted that engagement does not require a direct connection (e.g., a direct physical or electrical connection), but rather that many objects or components may be used to engage two or more objects. For example, objects A and B may be engaged using object C. Furthermore, the terms "detachably connected" or "detachably engaged" may be interpreted as meaning a non-permanent connection or engagement between two or more objects. This means that detachably connected objects may be unconnected and separated, allowing them to operate without being physically joined.

Finally, the terms "or" and "and/or" as used herein should be interpreted inclusively, meaning either one or any combination thereof. Therefore, "A, B, or C" or "A, B, and/or C" means any one of the following: A; B; C; A and B; A and C; B and C; A, B, and C. Exceptions to this definition will only occur when the combination of elements, functions, steps, or actions is inherently mutually exclusive in some way.

### Overview of Master-Slave Teleoperated Laparoscopic Surgical Robots

Laparoscopic surgical robots typically consist of a surgeon control platform, a patient surgical platform, and an imaging platform. The surgeon sits on the surgeon control platform, viewing two-dimensional or three-dimensional images of the surgical area transmitted by a laparoscope placed inside the patient's body. The surgeon controls the movement of the robotic arm on the patient surgical platform, as well as the surgical instruments or laparoscopes attached to that arm. The robotic arm essentially simulates the human arm, and the surgical instruments simulate the human hand. The robotic arm and the surgical instruments provide the surgeon with a range of movements mimicking the human wrist while filtering out hand tremors.

The patient surgical platform includes a chassis, a column, robotic arms connected to the column, and one or more surgical instrument manipulators at the end of a support assembly of each robotic arm. Surgical instruments and/or endoscopes are detachably attached to the surgical instrument manipulator. Each surgical instrument manipulator supports one or more surgical instruments and/or endoscopes operating at the surgical site within the patient's body. The associated surgical instruments may be provided in various forms that allow each surgical instrument manipulator to move with one or more mechanical degrees of freedom (e.g., all six Cartesian degrees of freedom, five or fewer Cartesian degrees of freedom, etc.). Typically, each surgical instrument manipulator is restricted by mechanical or software constraints to rotate the associated surgical instrument about a center of motion on the surgical instrument that remains stationary relative to the patient. This center of motion is typically located at the point where the surgical instrument enters the body, and this center of motion is referred to as the "Remote Center of Motion".

The imaging platform typically includes a video image capture function (commonly an endoscope) and one or more video displays for showing surgical instruments in the captured images. In some laparoscopic surgical robots, the endoscope includes optics of one or more imaging sensors (e.g., CCD or CMOS sensors) that transmit images from the patient's body to the distal end of the endoscope. The video images are then transmitted to the host unit of the imaging platform after photoelectric conversion and other steps. Subsequently, image processing is performed, and the processed images are displayed on the video displays for the assistant to observe.

The surgeon control platform may be located at a single location within a surgical system comprised of laparoscopic surgical robots, or it may be distributed across two or more locations within the system. Remote master/slave operation may be performed according to a preset level of control. In some embodiments, the surgeon control platform includes one or more manually operated input devices, such as joysticks, exoskeleton gloves, power and gravity-compensated manipulators, etc. These input devices acquire the surgeon's operating signals, which are processed by the control system to generate control signals for the robotic arms and surgical instrument manipulators, thereby controlling the remote-controlled motors on the surgical instrument manipulators, which in turn control the movement of the surgical instruments.

Typically, the force generated by the remote-controlled motor is transmitted via a drive system to the end effector of the surgical instrument. In some remote surgical embodiments, the input device for controlling the manipulator may be located remotely from the patient, either inside or outside the patient's room, or even in a different city. The input signal from the input device is then transmitted to the control system. Those familiar with remote manipulation, remote control, and remote presentation surgery will understand such a system and its components.

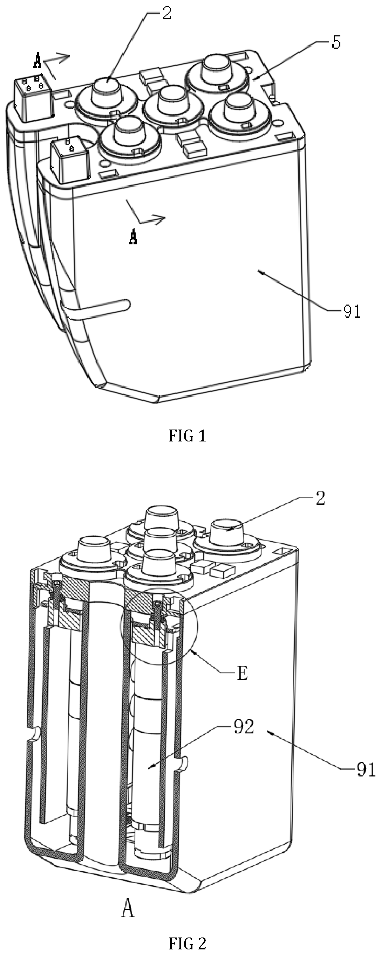

Surgical instrument manipulators typically drive the end-effectors to move via a power box mounted on them. As shown in FIGS 1 to 2, in some embodiments of the present disclosure, the power box may include a motor 92, a housing 91, and a power box output assembly. Multiple motors 92 may be disposed within the housing 91, and the motor shafts of the motors 91 are all connected to the power box output assembly.

In some examples, five motors 91 are provided. It is understood that the number of motors 91 in some examples of embodiments of the present disclosure is merely shown as a specific example. In other examples, there may be four or six motors 91, etc.

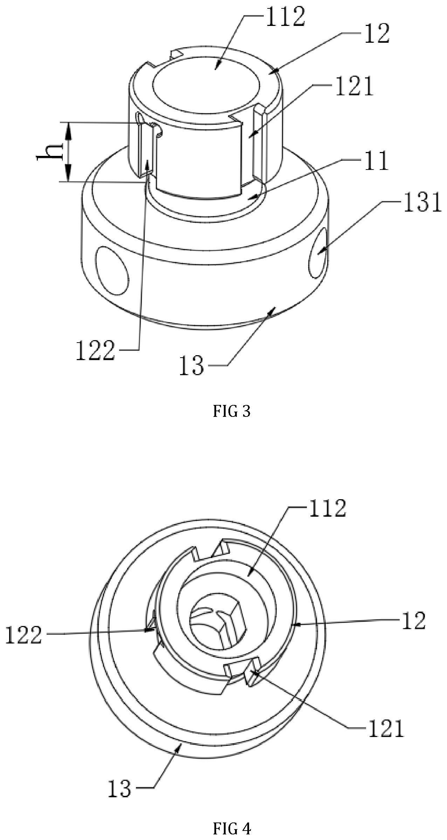

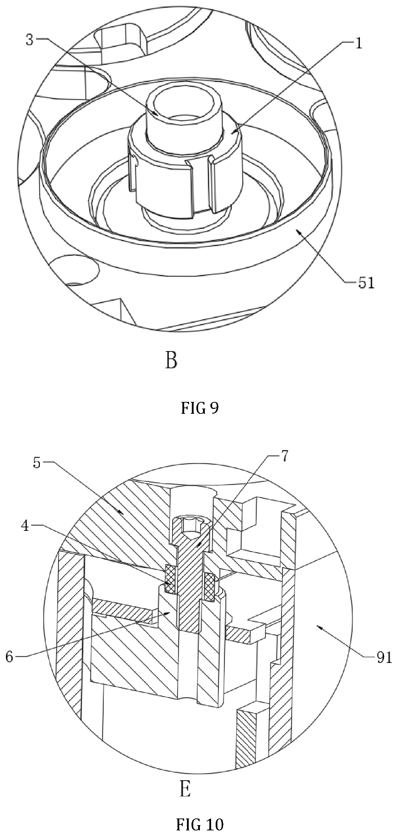

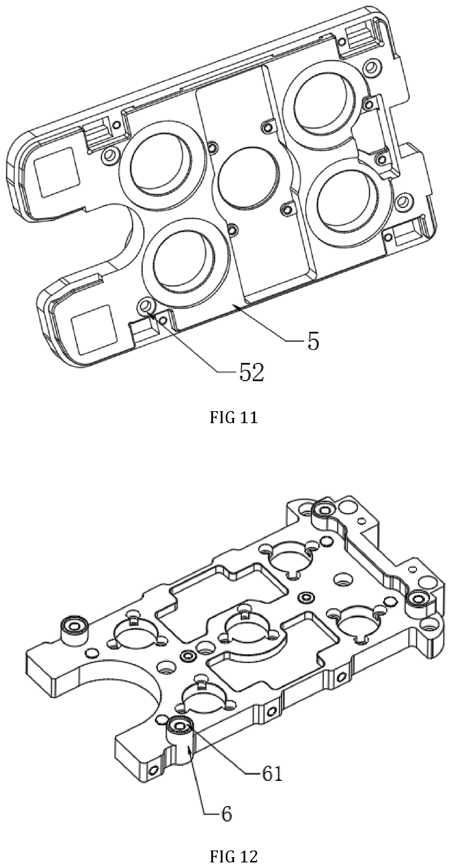

As shown in FIGS 3 to 12, in some examples of embodiments of the present disclosure, the power box output assembly may include a transmission member 1, an output member 2, and an elastic member 3. The motor shaft is detachably connected to the transmission member 1. For example, in some examples, the motor shaft of one motor 91 may be detachably connected to one transmission member 1.

In some examples, the output member 2 is mounted on the transmission member 1.

In some examples, the elastic member 3 is positioned between the transmission member 1 and the output member 2. One end of the elastic member 3 abuts the transmission member 1, and the other end of the elastic member 3 abuts the output member 2.

In some examples, the elastic member 3 is configured to push the output member 2 away from the transmission member 1.

In some examples, a limiting engagement mechanism may be provided between the transmission member 1 and the output member 2. The limiting engagement mechanism is configured to prevent the transmission member 1 and the output member 2 from separating axially. The limiting engagement mechanism is also configured to prevent the transmission member 1 and the output member 2 from rotating relative to each other circumferentially.

It should be noted that the transmission member 1 and the output member 2 may be assembled later. To facilitate the assembly of the transmission member 1 and the output member 2, they may generally be a clearance fit in the circumferential direction.

In other words, in some examples, the transmission member 1 and the output member 2 may rotate circumferentially within a preset angle range.

In some examples, the preset angle range may not exceed 1°.

It should be noted that this small range of rotation between the transmission member 1 and the output member 2 should not be considered a counterexample to the aforementioned "the limiting engagement mechanism being configured to prevent the transmission member 1 and the output member 2 from rotating relative to each other circumferentially". This rotation may be due to manufacturing errors or assembly requirements, and should fall within the scope claimed in the present disclosure. Such errors are negligible to those skilled in the art. Of course, if the clearance needs to be eliminated, a solution similar to that described in CN114533282B may be used.

In some examples, the limiting engagement mechanism uses surface-to-surface contact for position limiting, which reduces the performance requirements of the limiting engagement mechanism, helps to reduce costs, slows down the wear of the limiting engagement mechanism, ensures transmission accuracy, and reduces maintenance frequency.

In some examples, the output member 2 is formed with a cover 22, and at least one limiting protrusion 21 is formed on the inner side of the cover 22.

In some examples, the transmission member 1 is formed with a main shaft 11 and a first limiting ring 12. The main shaft 11 is configured to be fixedly connected to the motor shaft of the motor 92. The first limiting ring 12 is formed on the outer cylindrical surface of the main shaft 11.

In some examples, at least a set of slots is provided on the first limiting ring 12. The slot set may include a sliding slot 121. In some examples, the sliding slot 121 extends vertically through the first limiting ring 12.

In some examples, the slot set may include a limiting slot 122. The limiting slot 122 extends downward through the first limiting ring 12.

In some examples, the cover 22 is sleeved onto the first limiting ring 12, the limiting protrusion 21 corresponds to the set of slots, and the limiting slot 122 and the limiting protrusion 21 constitute the limiting engagement mechanism.

In some examples, the limiting protrusion 21 may correspond one-to-one with the slot set.

In some examples, the limiting protrusion 21 may slide linearly within the sliding slot 121.

In some examples, the limiting protrusion 21 may slide linearly within the limiting slot 122.

In some examples, one limiting protrusion 21 may be inserted into the sliding slot 121. One limiting protrusion 21 may be inserted into the limiting slot 122. The limiting protrusion 21 may slide linearly within the sliding slot 121 and the limiting slot 122.

In some examples, the elastic member 3 is disposed inside the cover 22, with the top end of the elastic member 3 abutting the top surface of the cover 22 and the bottom end abutting the main shaft 11.

It is understood that the positions of the slot set and the limiting protrusions 21 in the foregoing embodiments of the present disclosure are shown only as specific examples. In some examples, the positions of the limiting protrusions 21 and the slot set may be interchanged. For example, the slot set may be formed on the output member 2. The limiting protrusions 21 may be formed on the first limiting ring 12.

In some examples, a portion of multiple slot sets may be formed on the output member 2. Another portion of the multiple slot sets may be formed on the first limiting ring 12. A portion of multiple limiting protrusions 21 may be formed on the output member 2. Another portion of the multiple limiting protrusions may be formed on the first limiting ring 12.

In some examples, description is made with the example where the slot set is formed on the first limiting ring 12, and the limiting protrusion 21 is formed on the output member 2. The output member 2 is sleeved on the transmission member 1, so that the limiting protrusion 21 is inserted into the sliding slot 121. Then, the output member 2 is pressed down until the limiting protrusion 21 is below the first limiting ring 12, and then it is rotated until the limiting protrusion 21 is below the corresponding limiting slot 122. The output member 2 is then released, and the limiting protrusion 21 may abut the top surface of the limiting slot 122 under the action of the elastic member 3, thus completing the assembly of the output member 2 and the transmission member 1.

In some examples, the limiting protrusion 21 contacts the wall surfaces of the limiting slot 122 and the sliding slot 121, thereby achieving circumferential and axial constraint between the transmission member 1 and the output member 2. The limiting engagement mechanism has a simple structure, which greatly reduces production costs and assembly difficulty, and helps to improve the manufacturer's production efficiency.

In some examples, the height of the limiting slot 122 and the sliding slot 121 may be adjusted by milling the bottom surface of the first limiting ring 12 to change the thickness of the first limiting ring 12.

In some examples, multiple slot sets are provided, and multiple limiting protrusions 21 are provided corresponding to the multiple slot sets. The multiple slot sets are evenly distributed around the axis of the first limiting ring 12.

In some examples, multiple limiting engagement mechanisms are used to constrain the transmission member 1 at different positions around its circumference, reducing problems such as force imbalance, stress concentration, and low transmission safety. Furthermore, since the multiple limiting engagement mechanisms perform simultaneous constraint by surface-to-surface contact, the time required for assembling multiple limiting engagement mechanisms can be effectively saved.

In some examples, two slot sets are provided, and the limiting slot 122 and the sliding slot 121 in the same slot set have a first preset angle along the radial direction of the first limiting ring 12.

In some examples, the first preset angle may be 90°. With a 90° angle, the centers of the limiting slots 122 and sliding slots 121 between different slot sets may be symmetrically and evenly distributed, maximizing the distance between slots and ensuring structural strength. It should be noted that in some embodiments of the present disclosure, the specific degree of the first preset angle is merely an illustrative example and is not intended to limit the specific degree of the first preset angle.

It should be noted that the numerical values and ranges involved in some embodiments of the present disclosure are approximate values. Due to the influence of the manufacturing process, there may be a certain range of errors, which may be considered negligible by those skilled in the art.

In some examples, the main shaft 11 is provided with a motor shaft hole 111. The motor shaft hole 111 may be configured to receive a motor shaft.

In some examples, the axis of the motor shaft hole 111 may be collinear with the axis of the main shaft 11. The motor shaft hole 111 may include a blind hole or a through hole. The blind hole or through hole may be provided on the bottom surface of the main shaft 11.

In some examples, the transmission member 1 is provided with at least one threaded hole 131. The threaded hole 131 may penetrate the sidewall of the motor shaft hole 111.

In some examples, a limiting screw may be installed inside the threaded hole 131. The motor shaft is pressed against the motor shaft hole 111 by the limiting screw to fix the motor shaft inside the motor shaft hole 111.

In some examples of embodiments of the present disclosure, a plane parallel to its axis is formed on the motor shaft, the motor shaft hole 111 is matched with the shape of the motor shaft, and at least one limiting screw passes through its corresponding threaded hole 131 and abuts the plane.

In some examples, two threaded holes 131 may be provided, with the two threaded holes 131 distributed around the axis of the main shaft 11, the axes of the two threaded holes 131 intersecting at the center of the motor shaft and having a second preset angle.

In some examples, the second preset angle may be 120°. With a 120° angle, the two limiting screws may ensure the motor shaft is subjected to the most balanced force, and its stability will not be affected regardless of the direction of external force disturbance. It should be noted that in some embodiments of the present disclosure, the specific degree of the second preset angle is merely an illustrative example and is not intended to limit the degree of the second preset angle. In some examples of embodiments of the present disclosure, the axes of the two limiting screws intersect at the center of the motor shaft, allowing the limiting screws to lock together, thus improving the connection strength between the transmission member 1 and the motor shaft.

In some examples, the transmission member 1 may be formed with a second limiting ring 13. The second limiting ring 13 is formed on the outer cylindrical surface of the main shaft 11.

In some examples, the second limiting ring 13 may be located below the first limiting ring 12.

In some examples, the threaded hole 131 may penetrate the sidewalls of the second limiting ring 13 and the motor shaft hole 111.

In some examples of embodiments of the present disclosure, the second limiting ring 13 increases the contact area between the transmission member 1 and the limiting screw, thereby improving the connection strength between the limiting screw and the transmission member 1.

In some examples, the diameter of the second limiting ring 13 does not affect the diameter of the first limiting ring 12. The diameter of the first limiting ring 12 may be smaller than the diameter of the second limiting ring 13 to reduce the diameter of the first limiting ring 12 and the output member 2, thus meeting the requirement of a compact end effector of the surgical robot.

In some examples, the main shaft 11 may be provided with a mounting hole 112. The mounting hole may be configured to receive an elastic member 13.

In some examples, the mounting hole 112 extends upward through the main shaft 11 and is collinear with the axis of the main shaft 11.

In some examples, when the motor shaft hole 111 is a through hole, the diameter of the mounting hole 112 may be larger than the diameter of the motor shaft hole 111, so that the bottom end of the elastic member 3 may abut the bottom surface of the mounting hole 112, and the hole wall of the mounting hole 112 defines the mounting position and extension direction of the elastic member 3.

In some examples, the elastic member 3 may be a spring.

In some examples, the output assembly of the power box 10 may include a top plate 5, which is the housing top plate of the power box 10 and is detachably connected to other components in the housing 91 of the power box 10.

In some examples, the transmission member 1 passes through the top plate 5. When the output member 2 is in its lowest position, the bottom surface of the output member 2 abuts the top surface of the top plate 5.

In some examples of embodiments of the present disclosure, the limiting protrusion 21 may be prevented from sliding out of the limiting slot 122 by means of the cooperation between the top plate 5 and the bottom surface of the first limiting ring 12.

In some examples, the top plate 5 has multiple through holes into which a transmission member 1 may be inserted. The through holes may be configured to define the mounting position of the second limiting ring 13 on the top plate 5.

In some examples, the top surface of the second limiting ring 13 may be lower than the top surface of the top plate 5.

In some examples, up to five through holes may be provided. It is understood that the number of through holes in some examples of embodiments of the present disclosure is merely illustrative and not a limitation on the number of through holes. In some examples, there may be other numbers of through holes as well.

In some examples, the output member 2 may be formed with flange ring 23. The flange ring 23 is formed on the outer cylindrical surface of the cover 22.

In some examples, when the output member 2 is in its lowest position, the flange ring 23 abuts the top surface of the top plate 5, and the diameter of the cover 22 may be smaller than the diameter of the through hole on the top plate 5 to reduce the size of the output member 2.

In some examples, the output assembly of the power box 10 may include a fixing boss 6 and a support post 4, and multiple fixing bosses 6 and multiple support posts 4 may be provided.

In some examples, the position of the fixing boss 6 relative to the motor 92 may be fixed. The fixing boss 6 and the support post 4 may correspond to each other.

In some examples, the top surface of the support post 4 may be in contact with the top plate 5, and the bottom surface may be in contact with the fixing boss 6.

In some examples of embodiments of the present disclosure, multiple fixing bosses 6 may be formed on one support plate, and the motor 92 may pass through the support plate.

In some examples of embodiments of the present disclosure, when the output member 2 is assembled with the transmission member 1, the limiting protrusion 21 is located outside the limiting slot 122. At the moment, there are no support posts 4 between the top plate 5 and all the fixing bosses 6, ensuring that the limiting protrusion 21 may enter the limiting slot 122 from below the first limiting ring 12. After the output member 2 and the transmission member 1 are assembled, the limiting protrusion 21 is located in the limiting slot 122. Each support post 4 is placed between the top plate 5 and the corresponding fixing boss 6. The top surface of each support post 4 abuts the top plate 5, and the bottom surface abuts the corresponding fixing boss 21, so as to raise the top plate 5 and prevent the limiting protrusion 21 from sliding out of the limiting slot 122 from below the first limiting ring 12, thereby improving the reliability of the connection between the output member 2 and the transmission member 1.

In some examples, the inner diameter of the cover 22 may be equal to the outer diameter of the first limiting ring 12, thus limiting the interaction between the output member 2 and the transmission member 1 to only rotation and vertical movement. Additionally, the widths of the limiting slot 122 and the sliding slot 121 may be equal to the width of the limiting protrusion 21, thus confining the limiting protrusion 21 to move only vertically within the sliding slot 121 and the limiting slot 122. This arrangement ensures that after the output member 2 and the transmission member 1 are assembled, the output member 2 has only one degree of freedom, i.e., vertical movement, relative to the transmission member 1, improving the transmission accuracy of the output assembly of the power box 10.

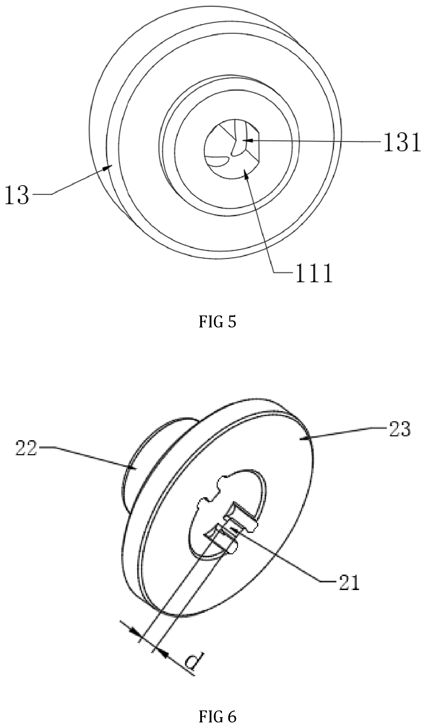

In some examples, as shown in FIG 3, the distance of the limiting slot 122 along the axial direction of the first limiting ring 12 is defined as h. As shown in FIG 6, the height of the limiting protrusion 21 is defined as d.

In some examples, in order to prevent the limiting protrusion 21 from disengaging from the limiting slot 122, it is necessary to ensure that after the output member 2 and the transmission member 1 are assembled, the height from the bottom surface of the flange ring 23 to the top surface of the top plate 5 is less than h. That is, the elastic member 3 holds the output member 2 at its highest point, meaning the travel distance of the output member 2 is less than h.

In some examples, it should be noted that when the power box output assembly provided in the foregoing embodiments of the present disclosure rotates, the force is transmitted through the surface-to-surface contact between the sidewall of the limiting slot 122 and the side edge of the limiting protrusion 21. Therefore, in order to ensure the reliability and lifespan of the power box output assembly, the travel distance of the output member 2 may be less than h-d. This ensures that the sidewall of the limiting protrusion 21 is fully accommodated within the limiting slot 122 throughout the travel range of the output member 2, thereby maintaining the maximum contact area between the sidewall of the limiting slot 122 and the side edge of the limiting protrusion 21.

In some examples, the top surface of the fixing boss 6 may have a first limiting hole 61, and the bottom surface of the top plate 5 may have a second limiting hole 52.

In some examples, the second limiting hole 52 may be set to correspond to the first limiting hole 61.

In some examples, one end of the support post 4 may be inserted into the first limiting hole 61. The other end of the support post 4 may be inserted into the second limiting hole 52.

In some examples, the outer diameter of the support post 4 is the same as the inner diameter of the first limiting hole 61 and the inner diameter of the second limiting hole 52. The cooperation between the first limiting hole 61, the second limiting hole 52 and the support post 4 limits the installation position of the top plate 5 and reduces the assembly difficulty.

In some examples, the output assembly of the power box 10 may include a fixing bolt 7. The fixing bolt 7 may correspond to the fixing boss 6, and the fixing bolt 7 may pass through the top plate 5 and the support post 4 and be threadedly connected to the fixing boss 6. The threaded connection simplifies the disassembly and assembly of the top plate 5, facilitating quick disassembly and assembly of the support post 4.

In some examples of embodiments of the present disclosure, four fixing bosses 6, four support posts 4, and four fixing bolts 7 are provided, and the four fixing bolts 7 are distributed in a matrix around the five motors 92.

In some examples, the output assembly of the power box 10 may include a third limiting ring 51, which corresponds to the output member 2.

In some examples, five third limiting rings 51 are provided.

In some examples, the third limiting ring 51 may be formed on the top surface of the top plate 5. The third limiting ring 51 may be configured to limit the installation position of the output member 2 on the top plate 5, reducing the assembly difficulty.

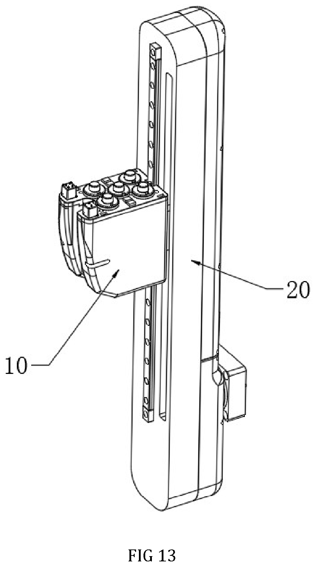

As shown in FIG 13, the embodiment of the present disclosure provides a slide carriage assembly, which may include a slide carriage 20 and the aforementioned power box 10. The power box 10 is mounted on the slide carriage 20, and the slide carriage 20 is provided with a drive mechanism for driving the power box 10 to slide linearly on the slide carriage 20. The power box output member in the power box 10 improves the transmission accuracy between the slide carriage assembly and the components connected to the power box.

The present disclosure provides a surgical robot, which may include a main control arm and a slave manipulator arm. The main control arm and the slave manipulator arm are signal-connected, and the end of the slave manipulator arm is the aforementioned slide carriage assembly. The power box 10 is detachably connected to an instrument box and transmits the rotational motion force of different motors 92 to the instrument box to improve the control accuracy of the motors 92 on the instrument box.

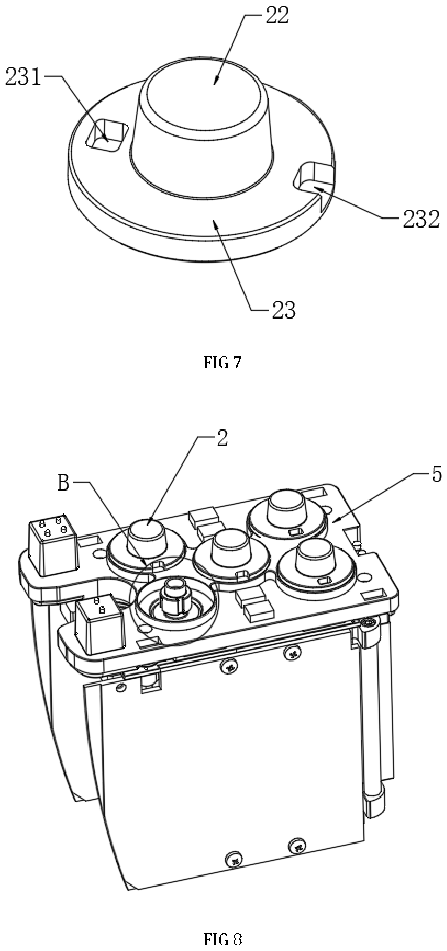

As shown in FIG 7, in this embodiment, the top surface of the flange edge may be provided with a positioning hole 231 and a notch 232. The positioning hole 231 and notch 232 may be asymmetrically distributed around the axis of the output member 2.

In some examples, the positioning hole 231 and the notch 232 may be connected to the isolation plate and similar components described in Chinese patent CN115630667A to transmit the rotational force of different motors 92 to the instrument box. Since the positions of the positioning hole 231 and the notch 232 are asymmetrical, the rotation angle of the output member 2 under the control of the motor shaft is uniquely determinable, making it easier to know the control position or posture of the end effector of the instrument on the instrument box.

In summary, in some embodiments of the present disclosure, the output member 2 and the transmission member 1 are circumferentially positioned relative to each other through the limiting engagement mechanism, and an elastic member 3 is provided between them to achieve floating docking. The structure is simple, the cost is low, and the assembly is easy and efficient.

The surface-to-surface contact method reduces wear between the output member 2 and the transmission member 1, while also ensuring the transmission accuracy between them. In addition, multiple limiting engagement mechanisms are provided around the circumference of the transmission member 1. Since the multiple limiting engagement mechanisms perform synchronous surface-to-surface contact positioning, it does not lead to an increase in assembly time.

In addition, in some examples of embodiments of the present disclosure, the top plate 5 is configured to limit the lowest position of the output member 2, and its height is adjustable, which can effectively prevent the limiting protrusion 21 from sliding out of the limiting slot 122 from below the first limiting ring 12, thereby improving the reliability of the connection between the output member 2 and the transmission member 1.

The above description is merely a preferred embodiment of the present disclosure and is not intended to limit the present disclosure. Various modifications and variations can be made to the present disclosure by those skilled in the art. Any modifications, equivalent substitutions, improvements, etc., made within the spirit and principles of the present disclosure should be included within the scope of the present disclosure.

## Claims

1. A power box output assembly, comprising a transmission member (1), an output member (2) and an elastic member (3), wherein:
the transmission member (1) is detachably and fixedly connected to the motor shaft of a motor (92), and the output member (2) is sleeved on the transmission member (1);
the elastic member (3) is disposed between the transmission member (1) and the output member (2), with one end abutting the transmission member (1) and the other end abutting the output member (2) to bias the output member (2) away from the transmission member (1); and
a limiting engagement mechanism is provided between the transmission member (1) and the output member (2) to prevent axial separation and circumferential relative rotation thereof.

2. The power box output assembly according to claim 1, wherein the transmission member (1) is provided with at least one slot set, with each slot set comprising a sliding slot (121) and a limiting slot (122), wherein the sliding slot (121) is open at both ends along the axial direction, and the limiting slot (122) is open at one end along the axial direction away from the output member (2); the output member (2) is formed with at least one limiting protrusion (21) corresponding one-to-one with the slot set; the limiting protrusion (21) is able to slide linearly in the corresponding sliding slot (121) or limiting slot (122); and the limiting slot (122) and the limiting protrusion (21) constitute the limiting engagement mechanism.

3. The power box output assembly according to claim 2, wherein the transmission member (1) is formed with a main shaft (11) and a first limiting ring (12), the main shaft (11) is detachably connected to the motor shaft of the motor (92); the first limiting ring (12) is formed on the outer cylindrical surface of the main shaft (11), and the output member (2) is sleeved on the first limiting ring (12); the sliding slot (121) and the limiting slot (122) are both provided on the first limiting ring (12); the limiting protrusion (21) is formed on the inner side of the output member (2); and the output member (2) is configured to be able to rotate around the axis of the transmission member (1) when the limiting protrusion (21) is located below the corresponding sliding slot (121) and limiting slot (122).

4. The power box output assembly according to claim 3, wherein multiple slot sets are provided, multiple limiting protrusions (21) are provided corresponding to the multiple slot sets, and all slot sets are evenly distributed around the axis of the first limiting ring (12).

5. The power box output assembly according to any one of claims 1 to 4, further comprising a top plate (5), the top plate (5) being the top plate of a housing of the power box, wherein the top plate (5) is detachably connected to other components of the housing of the power box; the transmission member (1) passes through the top plate (5); and a bottom surface of the output member (2) abuts a top surface of the top plate (5) when the output member (2) is in the lowest position.

6. The power box output assembly according to claim 5, further comprising multiple fixing bosses (6) and multiple support posts (4), wherein the fixing boss (6) is fixed relative to the motor (92), and the fixing boss (6) and the support post (4) correspond to each other; the top surface of the support post (4) abuts the top plate (5), and the bottom surface abuts the corresponding fixing boss (6) to constrain the limiting protrusion (21) in the limiting slot (122).

7. The power box output assembly according to claim 6, wherein a first limiting hole (61) is provided on a top surface of the fixing boss (6), and a second limiting hole (52) is provided on a bottom surface of the top plate (5) corresponding to the first limiting hole (61); one end of the support post (4) is inserted into the first limiting hole (61), and the other end of the support post (4) is inserted into the second limiting hole (52), and the outer diameter of the support post (4) is the same as the inner diameter of the first limiting hole (61) and the inner diameter of the second limiting hole (52).

8. The power box output assembly according to claim 6, further comprising a fixing bolt (7) corresponding to the fixing boss (6), wherein the fixing bolt (7) passes through the top plate (5) and the support post (4) and is threadedly connected to the fixing boss (6).

9. The power box output assembly according to claim 5, wherein the output member (2) is formed with a cover (22) and a flange ring (23), the flange ring (23) is formed on the outer cylindrical surface of the cover (22), the limiting protrusion (21) is formed on the inner side of the cover (22), and the cover (22) is sleeved on the first limiting ring (12).

10. The power box output assembly according to claim 3, further comprising a limiting screw; the main shaft (11) is provided with a motor shaft hole (111), the axis of the motor shaft hole (111) is collinear with the axis of the main shaft (11), and the motor shaft hole (111) is a blind hole or through hole located on a bottom surface of the main shaft (11); the transmission member (1) is provided with at least one threaded hole (131) penetrating the side wall of the motor shaft hole (111), and one limiting screw is provided in each threaded hole (131).

11. The power box output assembly according to claim 10, wherein two threaded holes (131) are provided, the two threaded holes (131) are distributed around the axis of the main shaft (11), and the axes of the two threaded holes (131) intersect.

12. The power box output assembly according to claim 10, wherein the transmission member (1) is further formed with a second limiting ring (13), the second limiting ring (13) being located below the first limiting ring (12) and formed on the outer cylindrical surface of the main shaft (11); the threaded hole (131) penetrates the sidewalls of the second limiting ring (13) and the motor shaft hole (111).

13. The power box output assembly according to claim 3, wherein a top surface of the main shaft (11) is provided with a mounting hole (112), a bottom end of the elastic member (3) abuts a bottom surface of the mounting hole (112), and a top end thereof abuts an inner top surface of the output member (2).

14. A power box, comprising a motor (92), a housing (91), and the power box output assembly according to any one of claims 1 to 13, wherein multiple motors (92) are disposed within the housing (91), and the motor shafts of the motors (92) are connected to the transmission member (1).

15. A slide carriage assembly, comprising a slide carriage (20) and the power box (10) according to claim 14; wherein the power box (10) is mounted on the slide carriage (20), and the slide carriage (20) is provided with a drive mechanism configured to drive the power box (10) to slide linearly on the slide carriage (20).

16. A surgical robot, comprising a master control arm and a slave manipulator arm, wherein the master control arm is signal-connected to the slave manipulator arm, and an end of the slave manipulator arm is the slide carriage assembly according to claim 15; the power box (10) is detachably connected to an instrument box and transmits the rotational motion force of different motors (92) to the instrument box.
